# EUROPEAN PATENT SPECIFICATION

(11) **EP 2 680 820 B1**
(45) Date of publication and mention of the grant of the patent: **02.11.2022**
(21) Application number: 12707954.9
(22) Date of filing: 01.03.2012
(51) Int. Cl.: A61K 9/127

(54) **ADVANCED ACTIVE LIPOSOMAL LOADING OF POORLY WATER-SOLUBLE SUBSTANCES**
FORTSCHRITTLICHES, AKTIVES LIPOSOMALES LADEN VON SCHWER WASSERLÖSLICHEN SUBSTANZEN
CHARGEMENT LIPOSOMAL ACTIF AVANCÉ DE SUBSTANCES MÉDIOCREMENT SOLUBLES DANS L'EAU

(30) Priority: 01.03.2011 NL 2006324; 01.03.2011 US 201161447770 P
(43) Date of publication of application: 08.01.2014
(73) Proprietor: 2-BBB Medicines B.V., 2333 CH Leiden (NL)
(72) Inventor: GAILLARD, Pieter Jaap, NL-2311 KE Leiden (NL); APPELDOORN, Chantal Catharina Maria, NL-2324 LD Leiden (NL); RIP, Jacob, NL-2313 NA Leiden (NL)
(74) Representative: Nederlandsch Octrooibureau
(86) International application number: PCT/NL2012/050123
(87) International publication number: WO 2012/118376

(56) References cited:
- WO-A1-2006/079216
- US-A1- 2005 025 822
- US-A1- 2005 106 231
- DOS SANTOS N ET AL: "pH gradient loading of anthracyclines into cholesterol-free liposomes: enhancing drug loading rates through use of ethanol", BIOCHIMICA ET BIOPHYSICA ACTA. BIOMEMBRANES, AMSTERDAM, NL, vol. 1661, no. 1, 10 February 2004 (2004-02-10), pages 47-60, XP004489710, ISSN: 0005-2736, DOI: 10.1016/J.BBAMEM.2003.11.016
- PIEL G ET AL: "Betamethasone-in-cyclodextrin-in-liposome : The effect of cyclodextrins on encapsulation efficiency and release kinetics", INTERNATIONAL JOURNAL OF PHARMACEUTICS, ELSEVIER, NL, vol. 312, no. 1-2, 7 April 2006 (2006-04-07), pages 75-82, XP027972695, ISSN: 0378-5173 [retrieved on 2006-04-07]

## Description

### FIELD OF THE INVENTION

The present invention relates to the field of encapsulation of substances with low aqueous solubility in liposomes. More in particular, it relates to active loading of poorly soluble drugs into liposomes. Liposomal compositions with high drug-load are provided by the present invention as well.

### BACKGROUND OF THE INVENTION

Effective delivery of diagnostic and therapeutic agents to cells and tissues is highly desirable in both medical and research settings. Although many delivery systems for diagnostic and therapeutic agents have been generated over the years, an effective delivery system for compounds with poor aqueous solubility having minimal side effects and low toxicity has remained elusive. Thus, there is a continuing need for diagnostic and therapeutic agent delivery technologies which achieves these goals.

The brain is an exceptionally challenging target for medical treatment and diagnosis. In particular, because the blood-brain barrier (BBB) inhibits the effectiveness of systemic administration of diagnostic and therapeutic agents to the central nervous system (CNS). Most drugs capable of crossing the BBB as well as many pharmacologically interesting compounds are lipophilic and consequently have a very low aqueous solubility or are even insoluble in water. The limited aqueous solubility prevents administration of the drug or functional compound intravenously. Additionally, administration via the blood may result in toxic side effects in the body. Furthermore, it is questionable whether the drug or functional compound reaches the target organ in a therapeutic amount. Compositions and methods for effective delivery of therapeutic and/or diagnostic compounds across the BBB to a CNS target are still needed.

It has been suggested that liposomes can enhance drug delivery to the brain across the BBB (EP 0652775 B1 and references therein). It is generally known to those skilled in the art that liposomes can be loaded with various components, including drugs, peptides and proteins. The advantages of liposomes for delivery of drugs and other functional compounds are primarily the reduced toxic side effects exhibited by liposomal formulations compared to administering the free compounds intravenously.

In utilizing liposomes for delivery of functional compounds, it is generally desirable to load the liposomes to high concentration, resulting in a high functional-compound-lipid mass ratio, since this reduces the amount of liposomes to be administered per treatment to attain the required therapeutic effect, all the more since several lipids used in liposomes have a dose-limiting toxicity by themselves (ref). The loading percentage is also of importance for cost efficiency, since poor loading results in a great loss of the active compound.

A variety of loading methods for encapsulating functional compounds, particularly drugs, in liposomes is available. Hydrophilic compounds for example can be encapsulated in liposomes by hydrating a mixture of the functional compounds and vesicle-forming lipids. This technique is called 'passive loading'. The functional compound is encapsulated in the liposome as the nanoparticle is formed. Lipophilic and to a lesser extent amphiphilic functional compounds are loaded somewhat more efficiently than hydrophilic functional compounds because they partition in both the lipid bilayer and the intraliposomal aqueous medium. However, using passive loading, the final functional-compound-to-lipid ratio as well as the encapsulation efficiency are generally low. The concentration of drug in the liposome equals that of the surrounding fluid and free non-entrapped drug is washed away after encapsulation.

The final functional-compound-to-lipid ratio of passive-loading techniques can be increased using solubility enhancers to increase the concentration of the lipophilic compound in the extraliposomal aqueous medium. Many methods, based on the use of co-solvents, surfactants, and complexing agents, for solubilizing lipophilic compounds in water have been developed. Several commonly used solubility enhancers in aqueous drug formulations are cyclodextrins, propylene glycol, polyethylene glycols, ethanol, sorbitol, nonionic surfactants, and polyethoxylated castor oil. Extreme pH may also be used in some cases to enhance solubility of poorly soluble drugs. These solubility enhancers and solubility enhancing conditions, however, can be toxic or irritating towards humans.

An overview of cyclodextrin-drug complexation is given by Challa et al., 2005. McCormack et al., 1994 & 1998, describe a method for entrapment of hydroxypropyl-β-cyclodextrin-drug inclusion complexes into liposomes with a passive-loading technique. The modified cyclodextrin acts as a solubility enhancer for the water-insoluble drugs dexamethasone, dehydroepiandrosterone, retinol, and retinoic acid, which results in liposomes with an increased drug-to-lipid mass ratio as compared to liposomes attained when loaded with uncomplexed drugs.

US 2009/0196918 A1 discloses liposomal formulations with inclusion complexes of hydroxypropyl-β-cyclodextrin or sulfobutylether-β-cyclodextrin and hydrophobic lactone drugs. The cyclodextrin-drug inclusion complex is entrapped into the liposomes via passive loading.

US 2007/0014845 A1 discloses a liposomal delivery vehicle, including a lipid derivatized with a hydrophilic polymer, for hydrophobic drugs with a typical aqueous solubility of less than about 50 µg/ml. Solubility of the hydrophobic drug inside the liposome is enhanced by formation of drug-cyclodextrins inclusion complexes. The cyclodextrin concentration inside the liposome preferably is above 400 mg/ml. Encapsulation of inclusion complexes takes place via passive loading.

Piel et al., 2006 discloses betamethasone-loaded liposome compositions, in which the encapsulation efficiency is enhanced by loading the liposome with betamethasone-cyclodextrin inclusion complexes.

Drawbacks of these passive-loading techniques are the undesired administering of solubility enhancer cyclodextrin in addition to the required functional compound as well as the fact that the encapsulation efficiency is still unsatisfactory.

Certain hydrophilic or amphiphilic compounds can be loaded into preformed liposomes using transmembrane pH- or ion-gradients (Zucker et al., 2009). This technique is called active or remote loading. Compounds amenable to active loading should be able to change from an uncharged form, which can diffuse across the liposomal membrane, to a charged form that is not capable thereof. Typically, the functional compound is loaded by adding it to a suspension of liposomes prepared to have a lower outside/higher inside pH- or ion-gradient. Via active loading, a high functional-compound-to-lipid mass ratio and a high loading efficiency (up to 100 %) can be achieved. Examples are active loading of anticancer drugs doxorubicin, daunorubicin, and vincristine (Cullis et al., 1997, and references therein)_.

US 2005/025822 A1 discloses an ammonium sulfate gradient-dependent active loading of PEGylated liposomes with a drug ciprofloxacin added in 10% sucrose solution for incubation with the liposomes.

WO 2006/079216 A1 discloses econazole-loaded liposomes made by liposome-micelle fusion, wherein econazole is first incorporated in micelles of PEGylated lipids which are subsequently combined with liposomes.

For lipophilic compounds having a non-polar/polar surface area ratio > 2.31, it is necessary that the drug has a reasonable solubility, >1.9 mM, in order to achieve effective active loading, because only soluble uncharged molecules can enter the liposome (Zucker et al., 2009). This implies that many lipophilic functional compounds, for example drugs relevant to crossing the BBB, do not result in sufficiently high functional-compound-to-lipid mass ratios via active loading.

### SUMMARY OF THE INVENTION

The present inventors have surprisingly found that efficient active loading of liposomes with high liposome loading can be achieved by first solubilizing a functional substance, which is itself insoluble in water, or otherwise has a very low aqueous solubility, using a solubility enhancer. The invention thus increases the functional-substance-to-lipid mass ratio of substances with low-aqueous solubility to levels above those attained by conventional active or passive loading techniques of liposomes and enlarges the range of functional substances, including drugs, amenable to encapsulation in liposomes.

Without wishing to be bound by any theory or to limit the scope of the invention, it is believed that the invention allows for the loading of liposomes with the solubility enhancer remaining outside of the liposome during and after loading. After separating off the liposomes from the fluid phase comprising non-entrapped functional compound typically very little or no solubilizer at all will be present in the remaining liposomal formulation.

As will be immediately apparent to those skilled in the art, the utility of the present process is not limited to pharmaceutical products. Any product or process requiring liposomal encapsulation of a poorly-water soluble substance can benefit from the present invention. Therefore, even though all embodiments specifically exemplified and/or defined herein concern pharmaceutical products, embodiments not relating to pharmaceutical products are also encompassed by this application.

### DETAILED DESCRIPTION OF THE INVENTION

As first aspect of the present invention concerns a method of loading liposomes with a poorly water-soluble substance, said poorly watersoluble substance having an aqueous solubility of less than 1.9 mM at 20 °C and pH = 7, said method comprising:
a) providing a mixture containing:
   i. liposomes encapsulating an internal aqueous medium;
   ii. said poorly water-soluble substance;
   iii. an external aqueous medium optionally comprising sucrose; and
   iv. a solubility enhancing agent a solubility enhancing agent present in the external aqueous medium, said solubility enhancing agent being selected from the group consisting of complexing agents chosen from α-, β-, and γ-cyclodextrin and cyclodextrins modified with alkyl-, hydroxyalkyl-, dialkyl, and sulphoalkylether groups, and co-solvents chosen fromand polyethylene glycols (PEGs);
   wherein a proton- and/or ion-gradient exists across the liposomal membrane; and
b) incubating said mixture for a period of time sufficient to cause at least part of said poorly water-soluble substance to be drawn out of the external aqueous medium and to accumulate in the liposomes under the influence of said proton and/or ion gradient; and
c) separating the liposomes from the external aqueous medium comprising the remaining poorly water-soluble substance and the solubility enhancing agent.

In this document and in its claims, the verb 'to comprise' and its conjugations are used in their non-limiting sense to mean that items following the word are included, without excluding items not specifically mentioned. In addition, reference to an element by the indefinite article 'a' or 'an' does not exclude the possibility that more than one of the element is present, unless the context clearly requires that there be one and only one of the elements. The indefinite article 'a' or 'an' thus usually means 'at least one'.

### Liposomes

The present method comprises providing a mixture containing pre-formed liposomes encapsulating an internal aqueous medium. The term 'liposome' is used herein in accordance with its usual meaning, referring to microscopic lipid vesicles composed of a bilayer of phospholipids or any similar amphipathic lipids encapsulating an internal aqueous medium. The liposomes can be unilamellar vesicles such as small unilamellar vesicles (SUVs) and large unilamellar vesicles (LUVs), and multilamellar vesicles (MLV), typically varying in size from 50 nm to 200 nm. No particular limitation is imposed on the liposomal membrane structure.

The term 'liposomal membrane' refers to the bilayer of phospholipids separating the internal aqueous medium from the external aqueous medium.

The liposomal membranes useful in the current invention may be formed from a variety of vesicle-forming lipids, typically -including dialiphatic chain lipids, such as phospholipids, diglycerides, dialiphatic glycolipids, single lipids such as sphingomyelin and glycosphingolipid, cholesterol and derivates thereof, and combinations thereof. As defined herein, phospholipids are amphiphilic substances having hydrophobic groups formed of long-chain alkyl chains, and a hydrophilic group containing a phosphate moiety. The group of phospholipids includes phosphatidic acid, phosphatidyl glycerols, phosphatidylcholines, phosphatidylethanolamines, phosphatidylinositols, phosphatidylserines, and mixtures thereof. Preferably, the phospholipids are chosen from 1,2-dipalmitoyl-sn-glycero-3-phosphocholine (DPPC), dimyristoyl-phosphatidylcholine (DMPC), hydrogenated soy phosphatidylcholine (HSPC), soy phosphatidylcholine (SPC), distearoyl phosphatidylcholine (DSPC), egg yolk phosphatidylcholine (EYPC) or hydrogenated egg yolk phosphatidylcholine (HEPC).

Liposomal membranes according to the present invention may further comprises ionophores like nigericin and A23187.

It is preferred that the liposomal phase transition temperature is between 0°C and 100°C, preferably between 4°C and 65°C. The phase transition temperature is the temperature required to induce a change in the physical state of the lipids constituting the liposome, from the ordered gel phase, where the hydrocarbon chains are fully extended and closely packed, to the disordered liquid crystalline phase, where the hydrocarbon chains are randomly oriented and fluid. Above the phase transition temperature of the liposome, the permeability of the liposomal membrane increases. Choosing a high transition temperature, where the liposome would always be in the gel state, could provide a non-leaking liposomal composition, i.e. the concentration of the poorly water-soluble substance in the internal aqueous medium is maintained during exposure to the environment. Alternatively, a liposome with a transition temperature between the starting and ending temperature of the environment it is exposed to, would provide a means to releasing the poorly water-soluble substance when the liposome passes through its transition temperature. Thus, the process temperature for the active-loading technique typically is above the liposomal phase transition temperature to facilitate the active-loading process. As is generally known in the art, phase transition temperatures of liposomes can, among other parameters, be influenced by the choice of phospholipids and by the addition of steroids like cholesterol, lanosterol, cholestanol, coprostanol, ergosterol, and the like. Hence, a method according to any of the foregoing is provided in which the liposomes comprise one or more components selected from different phospholipids and cholesterol in several molar ratios in order to modify the transition, the required process temperature and the liposome stability in plasma. Less cholesterol in the mixture will result in less stable liposomes in plasma. A preferable phospholipids composition comprises between 10 and 50 mol% of steroids, preferably cholesterol.

In accordance with the invention, liposomes can be prepared by any of the techniques now known or subsequently developed for preparing liposomes. For example, the liposomes can be formed by the conventional technique for preparing multilamellar lipid vesicles (MLVs), that is, by depositing one or more selected lipids on the inside walls of a suitable vessel by dissolving the lipids in chloroform and then evaporating the chloroform, and by then adding the aqueous solution which is to be encapsulated to the vessel, allowing the aqueous solution to hydrate the lipid, and swirling or vortexing the resulting lipid suspension. This process engenders a mixture including the desired liposomes. Alternatively, techniques used for producing large unilamellar lipid vesicles (LUVs), such as reverse-phase evaporation, infusion procedures, and detergent dilution, can be used to produce the liposomes. A review of these and other methods for producing lipid vesicles can be found in the text Liposome Technology, Volume I, Gregory Gregoriadis Ed., CRC Press, Boca Raton, Fla., (1984).

For example, the lipid-containing particles can be in the form of steroidal lipid vesicles, stable plurilamellar lipid vesicles (SPLVs), monophasic vesicles (MPVs), or lipid matrix carriers (LMCs). In the case of MLVs, if desired, the liposomes can be subjected to multiple (five or more) freeze-thaw cycles to enhance their trapped volumes and trapping efficiencies and to provide a more uniform interlamellar distribution of solute.

Following liposome preparation, the liposomes may be sized to achieve a desired size range and relatively narrow distribution of liposome sizes. A size range of about 20-200 nanometers allows the liposome suspension to be sterilized by filtration through a conventional filter, typically a 0.22 or 0.4 micron filter. The filter sterilization method can be carried out on a high through-put basis if the liposomes have been sized down to about 20-200 nanometers. Several techniques are available for sizing liposomes to a desired size. Sonicating a liposome suspension either by bath or probe sonication produces a progressive size reduction down to small unilamellar vesicles less than about 50 nanometer in size. Homogenization is another method which relies on shearing energy to fragment large liposomes into smaller ones. In a typical homogenization procedure, multilamellar vesicles are recirculated through a standard emulsion homogenizer until selected liposome sizes, typically between about 50 and 500 nanometers, are observed. In both methods, the particle size distribution can be monitored by conventional laser-beam particle size determination. Extrusion of liposome through a small-pore polycarbonate membrane or an asymmetric ceramic membrane is also an effective method for reducing liposome sizes to a relatively well-defined size distribution Typically, the suspension is cycled through the membrane one or more times until the desired liposome size distribution is achieved. The liposomes may be extruded through successively smaller-pore membranes, to achieve a gradual reduction in liposome size. Other useful sizing methods such as sonication, solvent vaporization or reverse phase evaporation are known to those of skill in the art.

For use liposomes having a size of from about 50 nanometer to about 180 nanometer are preferred.

The 'internal aqueous medium', as referred to herein, typically is the original medium in which the liposomes were prepared and which initially becomes encapsulated upon formation of the liposome. Accordingly, freshly prepared liposomes encapsulating the original aqueous medium can be used directly for active loading. Embodiments are also envisaged however wherein the liposomes, after preparation, are dehydrated, e.g. for storage. In such embodiments the present process may involve addition of the dehydrated liposomes directly to the external aqueous medium used to create the transmembrane gradients. However it is also possible to hydrate the liposomes in another external medium first, as will be understood by those skilled in the art. Liposomes are preferably dehydrated under reduced pressure using standard freeze-drying equipment or equivalent apparatus. The liposomes and their surrounding medium can be frozen in liquid nitrogen before being dehydrated and placed under reduced pressure. Dehydration without prior freezing takes longer than dehydration with prior freezing, but the overall process is gentler without the freezing step, and thus there is subsequently less damage to the liposomes and a smaller loss of the internal contents. To ensure that the liposomes will survive the dehydration process without losing a substantial portion of their internal contents, one or more protective sugars are typically employed to interact with the lipid vesicle membranes and keep them intact as the water in the system is removed. A variety of sugars can be used, including such sugars as trehalose, maltose, sucrose, glucose, lactose, and dextran. In general, disaccharide sugars have been found to work better than monosaccharide sugars, with the disaccharide sugars trehalose and sucrose being most effective. Other more complicated sugars can also be used. For example, aminoglycosides, including streptomycin and dihydrostreptomycin, have been found to protect liposomes during dehydration. Typically, one or more sugars are included as part of either the internal or external media of the lipid vesicles. Most preferably, the sugars are included in both the internal and external media so that they can interact with both the inside and outside surfaces of the liposomes' membranes. Inclusion in the internal medium is accomplished by adding the sugar or sugars to the buffer which becomes encapsulated in the lipid vesicles during the liposome formation process. In these embodiments the external medium used during the active loading process should also preferably include one or more of the protective sugars

As is generally known to those skilled in the art, polyethylene glycol (PEG)-lipid conjugates have been used extensively to improve circulation times for liposome-encapsulated functional compounds, to avoid or reduce premature leakage of the functional compound from the liposomal composition and to avoid detection of liposomes by the body's immune system. Attachment of PEG-derived lipids onto liposomes is called PEGylation. Hence, in a particularly preferred embodiment the liposomes are PEGylated liposomes. PEGylation can be accomplished by incubating a reactive derivative of PEG with the target liposomes. Suitable PEG-derived lipids include conjugates of DSPE-PEG, functionalized with one of carboxylic acids, glutathione (GSH), maleimides (MAL), 3-(2-pyridyldithio) propionic acid (PDP), cyanur, azides, amines, biotin or folate, in which the molecular weight of PEG is between 2000 and 5000 g/mol. Other suitable PEG-derived lipids are mPEGs conjugated with ceramide, having either C₈ or C₁₆-tails, in which the molecular weight of mPEG is between 750 and 5000 g/mol. Still other appropriate ligands are mPEGs or functionalized PEGs conjugated with glycerophospholipds like 1,2-dimyristoyl-sn-glycero-3-phosphoethanolamine (DMPE), 1,2-dipalmitoyl-sn-glycero-3-phosphoethanolamine (DPPE), 1,2-dioleoyl-sn-glycero-3-phosphoethanolamine (DOPE) and 1,2-distearoyl-sn-glycero-3-phosphoethanolamine (DSPE), and the like. PEGylation of liposomes is a technique generally known by those skilled in the art.

In a particularly preferred embodiment the liposomes are PEGylated with DSPE-PEG-GSH conjugates (up to 5 mol %) and/or DSPE-mPEG conjugates (wherein the molecular weight of PEG is typically within the range of 750-5000 g/mol, e.g. 2000 g/mol). The phospholipids composition may comprise up to 5-20 mol % of PEG-lipid conjugates.

Furthermore, in certain embodiments, it is desirable to incorporate into the liposomal membrane moieties that specifically target the liposome to a particular cell type, tissue or the like. Targeting of liposomes using a variety of targeting moieties (e.g., ligands, receptors and monoclonal antibodies) has been previously described. Suitable examples of such targeting moieties include lipoprotein lipase (LPL), [alpha]2-macroglobulin ([alpha]2M), receptor associated protein (RAP), lactoferrin, desmoteplase, tissue- and urokinase-type plasminogen activator (tPA/uPA), plasminogen activator inhibitor (PAI-I), tPA/uPA:PAI-I complexes, melanotransferrin (or P97), thrombospondin 1 and 2, hepatic lipase, factor VIIa/tissue-factor pathway inhibitor (TFPI), factor Villa, factor IXa, A[beta]I-40, amyloid-[beta] precursor protein (APP), CI inhibitor, complement C3, apolipoproteinE (apoE), pseudomonas exotoxin A, CRM66, HIV-I Tat protein, rhinovirus, matrix metalloproteinase 9 (MMP-9), MMP-13 (collagenase-3), spingolipid activator protein (SAP), pregnancy zone protein, antithrombin III, heparin cofactor II, [alpha]I-antitrypsin, heat shock protein 96 (HSP-96), platelet-derived growth factor (PDGF), apolipoproteinJ (apoJ, or clusterin), A[beta] bound to apoJ and apoE, aprotinin, angiopep-2 (TFFYGGSRGKRNNFKTEEY), very-low-density lipoprotein (VLDL), transferrin, insulin, leptin, an insulin-like growth factor, epidermal growth factors, lectins, peptidomimetic and/or humanized monoclonal antibodies or peptides specific for said receptors (e.g., sequences HAIYPRH and THRPPMWSPVWP that bind to the human transferrin receptor, or anti-human transferrin receptor (TfR) monoclonal antibody A24), hemoglobin, non- toxic portion of a diphtheria toxin polypeptide chain, all or a portion of the diphtheria toxin B chain, all or a portion of a non-toxic mutant of diphtheria toxin CRM197, apolipoprotein B, apolipoprotein E (e.g., after binding to polysorb-80 coating), vitamin D-binding protein, vitamin A/retinol- binding protein, vitamin B12/cobalamin plasma carrier protein, glutathione and transcobalamin-B 12.

Targeting mechanisms generally require that the targeting agents be positioned on the surface of the liposome in such a manner that the target moieties are available for interaction with the target, for example, a cell surface receptor. The liposome is typically fashioned in such a way that a connector portion is first incorporated into the membrane at the time of forming the membrane. The connector portion must have a lipophilic portion which is firmly embedded and anchored in the membrane. It must also have a hydrophilic portion which is chemically available on the aqueous surface of the liposome. The hydrophilic portion is selected so that it will be chemically suitable to form a stable chemical bond with the targeting agent which is added later. Therefore, the connector molecule must have both a lipophilic anchor and a hydrophilic reactive group suitable for reacting with the target agent and holding the target agent in its correct position, extended out from the liposome's surface. Techniques for incorporating a targeting moiety in the liposomal membrane are generally known in the art.

### Poorly water-soluble substance

As indicated above, the present invention concerns loading of liposomes with a poorly water-soluble substance. In the context of the present invention the term 'poorly water soluble' means having an aqueous solubility of less than 1.9 mM at 20°C, and pH = 7, even more preferable having an aqueous solubility of less than 1 mM.

According to an embodiment of the invention, the poorly water-soluble substance is a medicinal compound selected from the group of steroids, anthracyclines, Paclitaxel, Rapamycin (Sirolimus), Diclofenac, Bupivacaine, Vincristine, Cetirizine, Fexofenadine, Primidone and other catecholamines, , salts and derivatives of these medicinal compounds and mixtures thereof.

This list of compounds, however, is not intended to restrict the scope of the invention. In fact, the functional compound can be any poorly water-soluble amphipathic weak base or amphipathic weak acid. As noted above, embodiments wherein the poorly water-soluble substance is not a pharmaceutical or medicinal substance are also envisaged.

In a preferred embodiment, the poorly water-soluble substance is a medicinal compound selected from the group of steroids, anthracyclines, Paclitaxel, Rapamycin (Sirolimus), Diclofenac, Crizotinib, quinolinic acid, PD 150606, Bupivacaine, Vincristine, Topotecan, Ciprofloxacin, Cetirizine, Fexofenadine, Primidone and other catecholamines, Epinephrine, salts and derivatives of these medicinal compounds and mixtures thereof.

Typically, poorly water-soluble amphipathic weak bases have an octanol-water distribution coefficient (logD) at pH 7 between -2.5 and 2 and pKa ≤ 11, while poorly water-soluble amphipathic weak acids have a logD at pH 7 between -2.5 and 2 and pKa > 3. Preferably, the poorly water-soluble substances to be actively loaded have good thermal stability (to about 70°C for 4 hours) and good chemical stability at higher (7-11) or lower (4-7) pH.

Typically, the terms 'weak base' and 'weak acid', as used in the foregoing, respectively refer to compounds that are only partially protonated or deprotonated in water. Examples of protonable agents include compounds having an aminogroup, which can be protonated in acidic media, and compounds which are zwitterionic in neutral media and which can also be protonated in acidic environments. Examples of deprotonable agents include compounds having a carboxygroup, which can be deprotonated in alkaline media, and compounds which are zwitterionic in neutral media and which can also be deprotonated in alkaline environments.

The term 'zwitterionic' refers to compounds that can simultaneously carry a positive and a negative electrical charge on different atoms. The term 'amphipathic', as used in the foregoing is typically employed to refer to compounds having both lipophilic and hydrophilic moieties. The foregoing implies that aqueous solutions of compounds being weak amphipathic acids or bases simultaneously comprise charged and uncharged forms of said compounds. Only the uncharged forms may be able to cross the liposomal membrane.

### Active Loading

As indicated above, the pre-formed liposomes are loaded with the poorly water-soluble substance according to an active or remote loading technique. The process of "active loading," involves the use of transmembrane potentials. The principle of active loading, in general, has been described extensively in the art. The terms 'active-loading' and 'remote-loading' are synonymous and will be used interchangeably.

During active loading, the poorly water-soluble substance is transferred from the external aqueous medium across the liposomal membrane to the internal aqueous medium by a transmembrane proton- or ion-gradient. The term 'gradient' of a particular compound as used herein refers to a discontinuous increase of the concentration of said compound across the liposomal membrane from outside (external aqueous medium) to inside the liposome (internal aqueous medium).

To create the concentration gradient, the liposomes are typically formed in a first liquid, typically aqueous, phase, followed by replacing or diluting said first liquid phase. The diluted or new external medium has a different concentration of the charged species or a totally different charged species, thereby establishing the ion- or proton-gradient.

The replacement of the external medium can be accomplished by various techniques, such as, by passing the lipid vesicle preparation through a gel filtration column, e.g., a Sephadex or Sepharose column, which has been equilibrated with the new medium, or by centrifugation, dialysis, or related techniques.

The efficiency of active-loading into liposomes depends, among other aspects, on the chemical properties of the substance to be loaded and the type and magnitude of the gradient applied. In an embodiment of the invention, a method as defined in any of the foregoing is provided employing a gradient across the liposomal membrane, in which said gradient is chosen from a pH-gradient, a sulphate-, phosphate-, citrate-, or acetate-salt gradient, an EDTA-ion gradient, an ammonium-salt gradient, an alkylated, e.g methyl-, ethyl-, propyl- and amyl, ammonium-salt gradient, a Mn²⁺-, Cu²⁺-, Na⁺-, K⁺-gradient, with or without using ionophores, or a combination thereof. These loading techniques have been extensively described in the art.

Preferably, the internal aqueous medium of pre-formed, i.e. unloaded, liposomes comprises a so-called active-loading buffer which contains water and, dependent on the type of gradient employed during active loading, may further comprise a sulphate-, phosphate-, citrate-, or acetate-salt, an ammonium-salt, an alkylated, e.g methyl-, ethyl-, propyl- and amyl, ammonium-salt, a Mn²⁺-, Cu²⁺- or Na⁺/K⁺-salt, an EDTA-ion salt, and optionally a pH-buffer to maintain a pH-gradient. The preferred concentration of salts in the internal aqueous medium of unloaded liposomes is between 1 and 1000 mM.

The external aqueous medium, used to establish the transmembrane gradient for active loading, comprises water, solubility enhancer, the poorly water-soluble substance(s) to be loaded, and optionally sucrose to adjust the osmolarity and/or a chelator like EDTA to aid ionophore activity, more preferably sucrose and/or EDTA.. Saline may also be used to adjust osmolarity. In a preferred embodiment of the invention a method for actively loading liposomes is provided wherein concentrations of the gradient-forming salt in the internal aqueous medium, and concentrations of the poorly water-soluble substance(s) and solubility enhancer in the external medium are established of such magnitude that net transport of the poorly water-soluble substance(s) across the liposomal membrane occurs during active loading.

In a more preferred embodiment the gradient is chosen from a pH-, ammonium sulphate and calcium acetate gradient. As is generally known by those skilled in the art, transmembrane pH- (lower inside, higher outside pH) or calcium acetate gradients can be used to actively load amphiphilic weak acids. Amphipathic weak bases can also be actively loaded into liposomes using an ammonium sulfate or ammonium chloride gradient. Unionized poorly water-soluble amphipathic compound crosses the membrane and is either protonized or deprotonized and/or forms an insoluble salt precipitate with the sulfate, chloride or calcium intraliposomal counter-ion.

Depending upon the permeability of the lipid vesicle membranes, the full transmembrane potential corresponding to the concentration gradient will either form spontaneously or a permeability enhancing agent, e.g., a proton ionophore may have to be added to the bathing medium. If desired, the permeability enhancing agent can be removed from the preparation after loading has been completed using chromatography or other techniques.

Typically the process temperature during active loading is between 0 and 100°C, preferably between 0 and 70°C, and most preferably between 4 and 65°C.

The encapsulation or loading efficiency, defined as encapsulated amount of moles of poorly water-soluble substance in the internal aqueous phase divided by the initial amount of moles of poorly water-soluble substance in the external aqueous phase multiplied by 100%, is at least 25 %, preferably at least 50%, at least 60%, or at least 70 %.

### Solubility enhancer

As noted herein before, it is the gist of the invention to add a solubility enhancer to the external aqueous medium to increase the rate and efficiency of uptake of the poorly soluble substance from the external medium into the liposome. Thereby, the concentration of dissolved poorly water-soluble substance in the external aqueous medium of the present invention is increased. According to the present invention, a method as defined in the foregoing is provided using a solubility enhancer chosen from certain complexing agents and co-solvents. The solubility enhancer typically increases the solubility of the poorly water-soluble compound in the external aqueous medium at least two-fold, preferably at least three-fold, preferably to values above 1.9 mM at ambient temperature, more preferably to values above 3.8 mM.

Complexing agents, as defined herein, are water-soluble compounds that form water-soluble inclusion complexes with the poorly water-soluble compound, hence increasing the aqueous solubility of the poorly water-soluble compound. Cyclodextrines are well known in the art for their ability to form stable noncovalent inclusion complexes with an large variety of amphiphilic and lipophilic guest molecules (Challa et al., 2005). They have an lipophilic inner cavity and a hydrophilic outer surface which amounts for their good aqueous solubility. The 3 naturally occurring cyclodextrins, α-, β-, and γ-cyclodextrin, differ in their ring size and aqueous solubility. Of these naturally occurring cyclodextrins, the lipophilic inner cavity of β-cyclodextrin is most suitable for a variety of functional compounds. Chemical modification with hydroxypropyl- and sulphoalkylether groups may increase the aqueous solubility and complexing activity of the naturally occurring cyclodextrins (Thompson 1997, Muller et al., 1985, Szente et al., 1999). In accordance with the invention, the complexing agent is chosen from α-, β-, and γ-cyclodextrin and cyclodextrins modified with alkyl-, hydroxyalkyl-, dialkyl, and sulphoalkylether groups.

In a preferred embodiment, the solubility enhancer is a complexing agent chosen from β-cyclodextrin, hydroxypropyl-β-cyclodextrin (HPβCD), and sulfobutylether-β-cyclodextrin. Preferably the complexing agent is present in the external aqueous medium in an amount of 0.1-25 w%, more preferably in an amount of 0.5-15 w% and most preferably in an amount of 1-12 w%, based on the total mass of the external aqueous medium.

As indicated above, the solubility enhancer can also be a co-solvent. The use of co-solvents to enhance the aqueous solubility of organic drug-like compounds is well known in the art (Sweetana et al., 1996; Rytting et al., 2005).

Co-solvents are water-miscible solvents that, once added to water, result in a mixture with, compared to pure water, greatly enhanced solubility for poorly water-soluble compounds.

In accordance with the invention, the co-solvent is chosen from the group of PEGs ,even more preferably the co-solvent is PEG with a molecular mass between 200 and 400 g/mol. Preferably the co-solvent is present in an amount of at least 1 wt%, more preferably at least 2 wt%, most preferably at least 5 wt%, based on the total mass of the external aqueous medium. Furthermore, it is preferred that the amount of co-solvent is less than 75 wt%, preferably less than 50 wt%, more preferably less than 33 wt% and most preferably less than 10 wt%, based on the total mass of the external aqueous medium.

In a most preferred embodiment, a method to actively load liposomes is provided, said method comprising the use of a solubility enhancer chosen from HPβCD, PEG with a molecular mass between 200 and 400 g/mol, , and mixtures thereof.

As noted herein before, the solubility enhancer that is present in the external aqueous medium preferably is not transferred across the liposomal membrane during active loading of the poorly water-soluble compound. Thus, according to a preferred embodiment an active-loading method according to any of the foregoing is provided, in which the solubility enhancer is not or only slightly able to pass the liposomal membrane, both physically and in time.

In a preferred embodiment, the solubility enhancing agent that is present in the external aqueous medium does not permeabilize the liposomal membrane.

In the event that the solubility enhancer is a complexing agent, it is preferred that the rate of dissociation of the complexing agent and the poorly water-soluble substance in the external medium exceeds the rate of uptake of the poorly-water soluble substance from the external medium into the liposome. Without wishing to be bound by any particular theory, it is believed that the former can be established by optimizing the concentrations and/or combinations of complexing agent and poorly water-soluble substance in the external medium as well as the proton- and/or ion-gradient across the liposomal membrane. Hence, in a preferred embodiment, a method for loading pre-formed liposomes according to any of the foregoing is provided wherein the processing temperature during active loading, the phase transition temperature of the liposomes, the concentrations and/or combinations of complexing agent and poorly water-soluble substance in the external medium as well as the proton- and/or ion-gradient across the liposomal membrane are optimized to such magnitude that the liposomal uptake of solubility enhancer is sufficiently low. Preferably, the concentration of solubility enhancer in the internal aqueous medium of the loaded liposome is lower than 20 mol %, based on the total number of moles of poorly water-soluble substance and solubility enhancer, more preferable the concentration of solubility enhancer in the loaded liposome is lower than 5 mol %, most preferably it is below 1 mol%.

As will be apparent from the foregoing, the rate and efficiency of active-loading a given poorly water-soluble substance into the liposome is affected by many factors, especially by the transmembrane gradient, the choice of solubility enhancing agent, the composition of the liposome membrane, the process temperature, etc. It is within the capabilities and the normal routine of those skilled in the art to adapt and optimize these parameters in conjunction to arrive at the most efficient process, i.e. for a given poorly water-soluble substance.

Regardless thereof, it is the merit of the present invention to have recognized for the first time that the use of a solubility enhancer in the external medium generally increases the rate of uptake as compared to the identical active loading process wherein no solubility enhancer is employed.

Hence, also disclosed is the use of a solubility enhancer as described in the foregoing in the active-loading of liposomes to enhance the loading efficiency and/or rate of poorly water-soluble substances. In a preferred embodiment said solubility enhancer is selected from the group consisting of complexing agents, co-solvents, surfactants, emulsifiers and combinations thereof. As will be understood, preferred embodiments involve the application of the pre-formed liposomes, poorly water-soluble substances, internal aqueous medium, external aqueous medium, gradients, etc. as defined in any of the foregoing. Preferably, the use comprises combining the solubility enhancing agent with the poorly water-soluble substance, a first aqueous medium (i.e. the external medium defined herein before) and liposomes encapsulating a second aqueous medium (i.e. the internal medium).

A further aspect of the present invention relates to a liposomal composition obtainable by the active-loading method described in the foregoing. As will be understood, preferred embodiments involve the liposomal compositions wherein the liposomes, the poorly water-soluble substances, internal aqueous medium, etc. are all as defined in any of the foregoing.

In a preferred embodiment of the invention, this composition has a poorly-water-soluble-substance-to-lipid mass ratio of at least 1:7, more preferably at least 1:5, and still more preferably at least 1:2.

Typically, said liposomal composition comprises the poorly water-soluble substances mainly in precipitated form, as will be understood by those skilled in the art. Typically no more than a minor fraction of the poorly water-soluble substance inside the liposomes is complexed with the solubility enhancer, e.g. less than 10 mol % and preferably less than 1 mol % of the poorly water-soluble compound is complexed with solubility enhancer in the internal aqueous medium.

Furthermore, In an embodiment, the amount of solubility enhancer in the internal aqueous medium of the loaded liposomes is lower than 20 mol %, relative to the total amount of poorly water-soluble substance and solubility enhancer, more preferable the concentration of solubility enhancer in the loaded liposome is lower than 5 mol %, most preferably it is below 1 mol%.

The invention is further illustrated by the following examples, which are by no means intended to limit the scope of the invention.

### EXAMPLES

### Example 1; Production of pre-formed active-loading liposomes.

Liposomes may consist of different phospholipids and cholesterol in several molar ratios in order to modify the transition/processing temperature and particle stability in plasma. Specifically, phospholipids like 1,2-dipalmitoyl-sn-glycero-3-phosphocholine (DPPC), dimyristoylphosphatidylcholine (DMPC), hydrogenated soy phosphatidylcholine (HSPC), soy phosphatidylcholine (SPC), distearoyl phosphatidylcholine (DSPC), or egg yolk phosphatidylcholine (EYPC) are used in different ratios with cholesterol (Chol), where less Chol in the mixture will result in less stable liposomes in plasma. Components are dissolved in ethanol or iso-propanol. Additionally, for the purpose of increasing the (in vivo) particle stability and enhanced brain delivery, micelles consisting of DSPE-PEG-GSH (up to 5 mol %), which is synthesized before preparation of the liposomes using DSPE-PEG-MAL and fresh solutions of reduced glutathione, and/or DSPE-mPEG (Mw 2000) may be added to the solution at different mol-percentages (up to 5-10 mol % in total). The (lipid) mixture is injected in aqueous solutions containing active loading buffer of different ionic strengths (1-1000 mM), e.g. 2 mg/mL ammonium sulfate or 1 mg/mL calcium acetate. After stirring, the formed vesicles are either extruded through membranes or homogenized in an emulsifier to create liposomes of a uniform size. Alternatively, DSPE-PEG-GSH or DSPE-mPEG is added after preparation of the liposomes by incubation at 25°C up to 60°C for 2 up to 24 hours (depending on the phase transition temperature of the lipid mixture and the temperature sensitivity of the agent). The liposomes are separated from the excess active loading buffer by means of general separation methods, like dialysis or diafiltration. Liposomes are characterized by measuring particle size (50-200 nm on a Malvern Zetasizer), zeta potential, phospholipid content (using the Phopholipids B kit or HPLC/UPLC systems) and peptide content (0.2-10 mol% GSH based on HPLC/UPLC or an OPA assay ).

### Example 2; Advanced active liposomal loading of poorly soluble compound A.

A small molecule (compound A) with a low molecular weight has an aqueous solubility of 0.003 mg/ml at ambient temperature (about 20°C). The solubility does not significantly increase at different pH (test range between pH 1.5 and 10), where in cyclodextrins, PEG₂₀₀ and nicotinamide, the solubility is increased to 2.5 mg/ml at pH = 7.

The compound is measured with UV at 262 nm at concentrations between 5-60 µg/ml or with a HPLC system equipped with a C-18 RP column.

The solubilized compound is exposed to a series of pre-formed liposomes as described in Example 1, in different drug to lipid ratio's (4:1, 2:1, 1:1, 1:2, 1:4) to determine the optimal encapsulation efficiency conditions.

Specifically, the optimal loading conditions are as follows. GSH-PEG-DSPE micelles (5%) are dissolved in a 2 mg/mL ammonium sulfate solution at 60°C. To this solution, HSPC (55%) and cholesterol (40%) dissolved in ethanol (at 60°C) is added and liposomes are prepared by extrusion through filters until particles of about 100 nm are obtained. Subsequently, free ammonium sulfate is removed by dialysis and stored at 4°C for further use. Then, a 2.5 mg/mL solution of compound A in 10% hydroxypropyl beta-cyclodextrin (HPβCD) is prepared and added to the pre-formed liposomes (at 60°C for 60 minutes), to allow for compound A to exchange with ammonium of the ammonium sulfate and precipitate within the liposome core. Directly after, the loaded liposomes are cooled down on ice and excess compound A in HPβCD is removed by dialysis, and stored at 4°C for further use. The initial drug to lipid ratio corresponding to the optimal loading conditions is 1:4.

The optimal encapsulation efficiency is 60-70% and the final product has liposomes containing 1 mg/ml compound A and a drug to lipid ratio of 1:5.7. This liposome solution can subsequently be concentrated 2-3 times, to render an even stronger solution. In contrast, compound A solubilized in PEG₂₀₀ and nicotinamide gives no active loading into the same pre-formed liposomes.

The loaded liposomes are tested for in vitro leakage in 50 mM phosphate buffer pH 7.4, 5% sucrose at 4°C and 37°C for 12 days (2, 4, 6, 24, 48, 120, 288 hours). Before the measurement, released compound is removed from the solution using size exclusion chromatography and the encapsulated compound is liberated with 50% 2-propanol and measured. From this analysis it is determined that some compound is liberated from the liposomes, similarly at both temperatures during the first 24 hours (approximately 20%), and that this leakage stabilizes after 24 hours.

From cell-based assays, it is know that compound A is a substrate for the drug efflux pump breast cancer resistance protein (BCRP, also known as ABCG2), so the formulations are tested both in wild-type and in BCRP knock-out mice. The compound is injected in the tail-vein of mice at a dose of 2.5 mg/kg. Animals are either injected with liposomal compound or free compound formulated in HPβCD. At 6 hours after injection, 52% of the injected dose of liposomal compound A is recovered in plasma and 0.5% in brain homogenates of wild type mice. In BCRP knock-out mice, these values are 50% in plasma and 0.6% in brain homogenates, respectively. In contrast, in wild-type animals these values are only 0.01% for the HPβCD formulation in plasma and 0.03% in brain homogenates, where BCRP knock-out mice have 0.23% of the injected dose of compound A formulated in HPβCD in the brain homogenates after 8 hours and in plasma the concentration is below the detection level.

In conclusion, by enhancing the solubility of compound A in HPβCD, a highly concentrated and stable liposomal formulation is prepared by an active-loading mechanism with an ammonium sulfate gradient. This liposomal formulation allows for a long blood circulating time and significantly enhances the brain delivery of compound A, to over twice the level that can be obtained in BCRP knock-out mice injected with the maximum tolerated amount of compound A formulated in HPβCD alone, and over 15 times the level that can be reached in wild type mice.

### Example 3; Advanced active liposomal loading of poorly soluble compound B.

A chemical molecule (compound B) is presented with a low molecular weight. The solubility in water is 0.048 mg/ml, and the compound decomposes at basic pH. The solubility is increased up to 8 mg/ml in PEG₂₀₀.

The compound is measured with UV at 295 nm at concentrations between 5-60 µg/ml. The solubilized compound is exposed to a series of pre-formed liposomes as described in example 1. Specifically, two different compositions with a favorable encapsulation efficiency are produced. Firstly, GSH-PEG-DSPE micelles (5%) are dissolved in a 250 mM calcium acetate solution at 60°C. To this solution, HSPC (55%) and cholesterol (40%) dissolved in ethanol (at 60°C) is added. Secondly, GSH-PEG-DSPE micelles (5%) are dissolved in a 250 mM calcium acetate solution at 25°C. To this solution, DMPC (55%) and cholesterol (40%) dissolved in ethanol (at 25°C) is added. For both compositions the liposomes are prepared by extrusion through filters until particles of about 100 nm are obtained. Subsequently, free calcium acetate is removed by dialysis and stored at 4°C for further use. Then, a 4 mg/mL solution of compound B in an (1:1 vol/vol)-mixture of PEG₂₀₀ and 5% sucrose in water is prepared and added to the pre-formed liposomes in different drug to lipid ratio's (at 60°C for the first composition and at 25°C for the second, both for 60 minutes), to allow for compound B to exchange with calcium acetate and precipitate within the liposome core. Directly after, the loaded liposomes are cooled down on ice and excess compound B in the PEG₂₀₀/sucrose mixture is removed by dialysis, and stored at 4°C for further use.

The optimal encapsulation efficiency of the first composition is 60-70% and at an initial drug to lipid ratio of 1:3. The final product has liposomes containing 1.8 mg/ml of compound B and a drug to lipid ratio of 1:4.5. For the second composition, the optimal encapsulation efficiency is 25-30% encapsulation at an initial drug to lipid ration of 1:3. The final product has liposomes containing 2 mg/ml of compound B and a drug to lipid ratio of 1:25. These liposome solutions can be concentrated 2-3 times, to render even stronger solutions. In contrast, compound B solubilized in pure PEG₂₀₀ and HPβCD gives no active loading into the same pre-formed liposomes, and also not in pre-formed liposomes containing ammonium sulfate.

The loaded liposomes are tested for in vitro leakage in 50 mM phosphate buffer pH 7.4, 5% sucrose at 4°C and 37°C on day 1, 3, and 7 after production. Before the measurement, released compound is removed from the solution using size exclusion chromatography and the encapsulated compound is liberated with 50% 2-propanol and measured. From this analysis it is determined that both formulations are stable in the refrigerator for at least a week and for the first composition for at least 1 day at 37°C. The second composition loses most of its compound after 1 day at 37°C.

The formulated compound is injected in the tail-vein of rats at a dose of 2 mg/kg. Animals are either injected with liposomal compound (composition 1 and 2) or free compound formulated in PEG₄₀₀-HPβCD (10:90 v/v). Both liposomal formulations have, however, a surprisingly short plasma half-life. Therefore, the assessment of distribution differences between the formulations is done at 15 minutes after injection. In these samples, 52% and 18% of the injected dose of liposomal compound B is recovered in plasma for composition 1 and 2, respectively, and 3.1% and 1.0% in brain homogenates, respectively. In contrast, these values are 7% for the PEG₄₀₀-HPβCD formulation in plasma and 2.8% in brain homogenates.

In conclusion, by enhancing the solubility of compound B in a 1:1 mixture of PEG₂₀₀ and 5% sucrose, highly concentrated and stable liposomal formulations are prepared by an active-loading mechanism using calcium acetate. These liposomal formulations have a short blood circulating time, albeit with higher plasma levels (Cₘₐₓ) than without liposomes directly after injection, and the liposomal formulations did not significantly enhance the brain delivery of compound B as compared to the already high brain levels obtained by the PEG₄₀₀-HPBCD formulation. Increasing the plasma stability of the liposomes, e.g. by reducing the calcium acetate buffer concentration, will further enhance the delivery of compound B to the brain.

### Example 4; Advanced active liposomal loading of poorly soluble compound C.

A blood-brain barrier impermeable agonist for the NMDA receptor (compound C), with a low molecular weight, has a pH-dependent solubility in water that ranges from 0.003 mg/mL at pH 5, 0.020 mg/mL at pH 6, 0.35 mg/mL at pH 7 to 0.9 mg/mL at pH 8. The solubility is further increased up to 2.2 mg/mL in HPβCD at pH = 7.

The compound is measured with UV at 262 nm at concentrations between 5-60 µg/ml or with an HPLC system equipped with a C-18 RP column.

The solubilized compound is exposed to a series of pre-formed liposomes as described in example 1, in different drug to lipid ratio's (4:1, 2:1, 1:1, 1:2, 1:4) to determine the optimal encapsulation efficiency conditions.

Specifically, the optimal loading conditions are as follows. GSH-PEG-DSPE micelles (5%) are dissolved in a 250 mM calcium acetate solution at 25°C. To this solution, DMPC (57%) and cholesterol (38%) dissolved in ethanol (at 25°C) is added and liposomes are prepared by extrusion through filters until particles of about 100 nm are obtained. Subsequently, free calcium acetate is removed by dialysis and stored at 4°C for further use. Then, a 2.2 mg/mL solution of compound C in an aqueous solution of 2 w/w% HPβCD is prepared and added to the pre-formed liposomes (at 25°C for 60 minutes), to allow for compound C to exchange with calcium of the calcium acetate and precipitate within the liposome core. Directly after, the loaded liposomes are cooled down on ice and excess compound C in HPβCD is removed by dialysis, and stored at 4°C for further use.

The encapsulation efficiency is 60-70% and the final product has liposomes containing 1 mg/ml compound C at a drug to lipid ration of 1:2. This liposome solution can be concentrated 3.5 times, to render an even stronger solution.

The compound is injected in the tail-vein of mice at a dose of 10 mg/kg. Animals are either injected with liposomal compound or free compound formulated in HPBCD. At 30 minutes after injection, 14% of the injected dose of liposomal compound C is recovered in plasma, 0.26% in brain homogenates and 0.09% in cerebral spinal fluid (CSF). In contrast, these values are 6% for the HPβCD formulation of compound C in plasma, 0.08% in brain homogenates and 0.03% in CSF. The effect of agonists on the NMDA receptor are biochemically assessed in the cerebellum of mice by quantification of the increase in cyclic guanosine 3',5'-monophosphate (cGMP) levels. At 30 minutes after injection, the HPβCD formulation of compound C gives no change in cGMP level, where the liposomal formulation gives a significant increase of 160% of the control level.

In conclusion, by enhancing the solubility of compound C in HPBCD, a highly concentrated liposomal formulation is prepared by an active-loading mechanism using a calcium acetate gradient. Despite the relatively short plasma half-life, this liposomal formulation significantly enhances the brain and CSF delivery of compound C, to over three times the level that can be obtained in mice injected with the maximum tolerated amount of compound C formulated in HPβCD alone at the same dose level, and over 15 times the level that can be reached with the maximum tolerated amount of the concentrated liposomal formulation. Increasing the plasma stability of the liposomes, e.g. by reducing the calcium acetate buffer concentration, will further enhance the delivery of compound C to the brain. The enhanced delivery to the brain obtained by this liposomal formulation is able to elicit a receptor-specific effect in the cerebellum that was not observed with the free compound.

### Example 5; Advanced active liposomal loading of poorly soluble Crizotinib.

A small molecule tyrosine kinase inhibitor (Crizotinib) with a low molecular weight of 450 Da has an aqueous solubility of < 0.1 mg/ml at ambient temperature (about 20°C). The solubility in hydroxypropyl beta-cyclodextrin (HPβCD) increases to 2.2 mg/ml, where in PEG₂₀₀ and nicotinamide, the solubility increases to at least 5 mg/ml at pH = 7.

The compound is measured with UV at concentrations between 5-60 µg/ml or with a HPLC system equipped with a C-18 RP column.

The solubilized compound is exposed to a series of pre-formed liposomes as described in Example 1, in different drug to lipid ratio's (4:1, 2:1, 1:1, 1:2, 1:4) to determine the optimal encapsulation efficiency conditions.

Specifically, the optimal loading conditions are as follows. GSH-PEG-DSPE micelles (5%) are dissolved in a 2 mg/mL ammonium sulfate solution at 60°C. To this solution, HSPC (55%) and cholesterol (40%) dissolved in ethanol (at 60°C) is added and liposomes are prepared by extrusion through filters until particles of about 100 nm are obtained. Subsequently, free ammonium sulfate is removed by dialysis and stored at 4°C for further use. Then, a 2.2 mg/mL solution of Crizotinib in 20% HPβCD is prepared and added to the pre-formed liposomes (at 60°C for 60 minutes), to allow for Crizotinib to exchange with ammonium of the ammonium sulfate and precipitate within the liposome core. Directly after, the loaded liposomes are cooled down on ice and excess Crizotinib in HPβCD is removed by dialysis, and stored at 4°C for further use. The initial drug to lipid ratio corresponding to the optimal loading conditions is 1:4.

The optimal encapsulation efficiency is 60-70% and the final product has liposomes containing 1 mg/ml Crizotinib and a drug to lipid ratio of 1:5.7. This liposome solution can subsequently be concentrated 2-3 times, to render an even stronger solution.

The loaded liposomes are tested for in vitro leakage in 50 mM phosphate buffer pH 7.4, 5% sucrose at 4°C and 37°C for 12 days (2, 4, 6, 24, 48, 120, 288 hours). Before the measurement, released compound is removed from the solution using size exclusion chromatography and the encapsulated compound is liberated with 50% 2-propanol and measured. From this analysis it is determined that some compound is liberated from the liposomes, similarly at both temperatures during the first 24 hours (approximately 20%), and that this leakage stabilizes after 24 hours.

From cell-based assays, it is know that tyrosine kinase inhibitors are often substrates for the drug efflux pump breast cancer resistance protein (BCRP, also known as ABCG2), so the formulations are tested both in wild-type and in BCRP knock-out mice. The compound is injected in the tail-vein of mice at a dose of 2.5 mg/kg. Animals are either injected with liposomal compound or free compound formulated in HPβCD. At 6 hours after injection, 52% of the injected dose of liposomal Crizotinib is recovered in plasma and 0.5% in brain homogenates of wild type mice. In BCRP knock-out mice, these values are 50% in plasma and 0.6% in brain homogenates, respectively. In contrast, in wild-type animals these values are only 0.01% for the HPβCD formulation in plasma and 0.03% in brain homogenates, where BCRP knock-out mice have 0.23% of the injected dose of Crizotinib formulated in HPβCD in the brain homogenates after 8 hours and in plasma the concentration is below the detection level.

In conclusion, by enhancing the solubility of Crizotinib in HPβCD, a highly concentrated and stable liposomal formulation is prepared by an active-loading mechanism with an ammonium sulfate gradient. This liposomal formulation allows for a long blood circulating time and significantly enhances the brain delivery of Crizotinib, to over twice the level that can be obtained in BCRP knock-out mice injected with the maximum tolerated amount of Crizotinib formulated in HPβCD alone, and over 15 times the level that can be reached in wild type mice.

### Example 6; Advanced active liposomal loading of poorly soluble PD 150606.

A calpain inhibitor (PD 150606) is presented, having a low molecular weight of 306 Da. The solubility in water is below 0.05 mg/ml, and the compound decomposes at basic pH. The solubility is increased up to 8 mg/ml in PEG₂₀₀.

The compound is measured with UV at concentrations between 5-60 µg/ml. The solubilized compound is exposed to a series of pre-formed liposomes as described in example 1. Specifically, two different compositions with a favorable encapsulation efficiency are produced. Firstly, GSH-PEG-DSPE micelles (5%) are dissolved in a 250 mM calcium acetate solution at 60°C. To this solution, HSPC (55%) and cholesterol (40%) dissolved in ethanol (at 60°C) is added. Secondly, GSH-PEG-DSPE micelles (5%) are dissolved in a 250 mM calcium acetate solution at 25°C. To this solution, DMPC (55%) and cholesterol (40%) dissolved in ethanol (at 25°C) is added. For both compositions the liposomes are prepared by extrusion through filters until particles of about 100 nm are obtained. Subsequently, free calcium acetate is removed by dialysis and stored at 4°C for further use. Then, a 4 mg/mL solution of compound B in an (1:1 vol/vol)-mixture of PEG₂₀₀ and 5% sucrose in water is prepared and added to the pre-formed liposomes in different drug to lipid ratio's (at 60°C for the first composition and at 25°C for the second, both for 60 minutes), to allow for PD 150606 to exchange with calcium acetate and precipitate within the liposome core. Directly after, the loaded liposomes are cooled down on ice and excess PD 150606 in the PEG₂₀₀/sucrose mixture is removed by dialysis, and stored at 4°C for further use.

The optimal encapsulation efficiency of the first composition is 60-70% and at an initial drug to lipid ratio of 1:3. The final product has liposomes containing 1.8 mg/ml of PD 150606 and a drug to lipid ratio of 1:4.5. For the second composition, the optimal encapsulation efficiency is 25-30% encapsulation at an initial drug to lipid ration of 1:3. The final product has liposomes containing 2 mg/ml of PD 150606 and a drug to lipid ratio of 1:25. These liposome solutions can be concentrated 2-3 times, to render even stronger solutions. In contrast, PD 150606 solubilized in pure PEG₂₀₀ and HPβCD gives no active loading into the same pre-formed liposomes, and also not in pre-formed liposomes containing ammonium sulfate.

The loaded liposomes are tested for in vitro leakage in 50 mM phosphate buffer pH 7.4, 5% sucrose at 4°C and 37°C on day 1, 3, and 7 after production. Before the measurement, released compound is removed from the solution using size exclusion chromatography and the encapsulated compound is liberated with 50% 2-propanol and measured. From this analysis it is determined that both formulations are stable in the refrigerator for at least a week and for the first composition for at least 1 day at 37°C. The second composition loses most of its compound after 1 day at 37°C.

The formulated compound is injected in the tail-vein of rats at a dose of 2 mg/kg. Animals are either injected with liposomal compound (composition 1 and 2) or free compound formulated in PEG₄₀₀-HPβCD (10:90 v/v). Both liposomal formulations have, however, a surprisingly short plasma half-life. Therefore, the assessment of distribution differences between the formulations is done at 15 minutes after injection. In these samples, 52% and 18% of the injected dose of liposomal PD 150606 is recovered in plasma for composition 1 and 2, respectively, and 3.1% and 1.0% in brain homogenates, respectively. In contrast, these values are 7% for the PEG₄₀₀-HPβCD formulation in plasma and 2.8% in brain homogenates.

In conclusion, by enhancing the solubility of PD 150606 in a 1:1 mixture of PEG₂₀₀ and 5% sucrose, highly concentrated and stable liposomal formulations are prepared by an active-loading mechanism using calcium acetate. These liposomal formulations have a short blood circulating time, albeit with higher plasma levels (Cₘₐₓ) than without liposomes directly after injection, and the liposomal formulations did not significantly enhance the brain delivery of PD 150606 as compared to the already high brain levels obtained by the PEG₄₀₀-HPBCD formulation. Increasing the plasma stability of the liposomes, e.g. by reducing the calcium acetate buffer concentration, will further enhance the delivery of PD 150606 to the brain.

### Reference

### Example 7; Advanced active liposomal loading of poorly soluble quinolinic acid.

A blood-brain barrier impermeable agonist for the NMDA receptor (quinolinic acid), with a low molecular weight of 167 Da, has a pH-dependent solubility in water that ranges from 0.003 mg/mL at pH 5, 0.020 mg/mL at pH 6, 0.35 mg/mL at pH 7 to 0.9 mg/mL at pH 8. The solubility is further increased up to 2.2 mg/mL in HPβCD at pH = 7.

The compound is measured with UV at 262 nm at concentrations between 5-60 µg/ml or with an HPLC system equipped with a C-18 RP column.

The solubilized compound is exposed to a series of pre-formed liposomes as described in example 1, in different drug to lipid ratio's (4:1, 2:1, 1:1, 1:2, 1:4) to determine the optimal encapsulation efficiency conditions.

Specifically, the optimal loading conditions are as follows. GSH-PEG-DSPE micelles (5%) are dissolved in a 250 mM calcium acetate solution at 25°C. To this solution, DMPC (57%) and cholesterol (38%) dissolved in ethanol (at 25°C) is added and liposomes are prepared by extrusion through filters until particles of about 100 nm are obtained. Subsequently, free calcium acetate is removed by dialysis and stored at 4°C for further use. Then, a 2.2 mg/mL solution of quinolinic acid in an aqueous solution of 2 w/w% HPβCDis prepared and added to the pre-formed liposomes (at 25°C for 60 minutes), to allow for quinolinic acid to exchange with calcium of the calcium acetate and precipitate within the liposome core. Directly after, the loaded liposomes are cooled down on ice and excess quinolinic acid in HPβCD is removed by dialysis, and stored at 4°C for further use.

The encapsulation efficiency is 60-70% and the final product has liposomes containing 1 mg/ml quinolinic acid at a drug to lipid ration of 1:2. This liposome solution can be concentrated 3.5 times, to render an even stronger solution.

The compound is injected in the tail-vein of mice at a dose of 10 mg/kg. Animals are either injected with liposomal compound or free compound formulated in HPBCD. At 30 minutes after injection, 14% of the injected dose of liposomal quinolinic acid is recovered in plasma, 0.26% in brain homogenates and 0.09% in cerebral spinal fluid (CSF). In contrast, these values are 6% for the HPβCD formulation of quinolinic acid in plasma, 0.08% in brain homogenates and 0.03% in CSF. The effect of agonists on the NMDA receptor are biochemically assessed in the cerebellum of mice by quantification of the increase in cyclic guanosine 3',5'-monophosphate (cGMP) levels. At 30 minutes after injection, the HPβCD formulation of quinolinic acid gives no change in cGMP level, where the liposomal formulation gives a significant increase of 160% of the control level.

In conclusion, by enhancing the solubility of quinolinic acid in HPBCD, a highly concentrated liposomal formulation is prepared by an active-loading mechanism using a calcium acetate gradient. Despite the relatively short plasma half-life, this liposomal formulation significantly enhances the brain and CSF delivery of quinolinic acid, to over three times the level that can be obtained in mice injected with the maximum tolerated amount of quinolinic acid formulated in HPβCD alone at the same dose level, and over 15 times the level that can be reached with the maximum tolerated amount of the concentrated liposomal formulation. Increasing the plasma stability of the liposomes, e.g. by reducing the calcium acetate buffer concentration, will further enhance the delivery of quinolinic acid to the brain. The enhanced delivery to the brain obtained by this liposomal formulation is able to elicit a receptor-specific effect in the cerebellum that was not observed with the free compound.

### REFERENCES

D. Zucker et al., Journal of Controlled Release, 2009 139 73-80
B. McCormack, G. Gregoriades, Journal of drug targeting, 1994 2(5) 449-454
B. McCormack, G. Gregoriades, Internationl Journal of Pharmaceutics, 1998 162 59-69
P.R. Cullis et al., Biochimica et Biophysica Acta 1997 1331 187-211
R. Challa et al., AAPS PharmSciTech 2005 6(2) E329-E357
J. Gubernator et al., Journal of Controlled Release 2010 146 68-75
A. Fritze et al, Biochimica et Biophysica Acta 2006 1758 1633-1640
A.S. Taggar et al., Journal of Controlled Release 2006 114 78-88
B.C.L. Cheung et al., Biochimica et Biophysica Acta 1998 1414 205-216
D.B. Fenske et al., Biochimica et Biophysica Acta 1998 1414 188-204
D.O. Thompson, CRC Crit. Rev. Ther. Drug Carrier Syst. 1997 14 1-104
B.W. Muller et al., Int. J. Pharm. 1985 26 77-88
L. Szente et al., Adv. Drug Deliv. Rev. 1999 36 17-38
S. Sweetana et al., PDA J. Pharm. Sci. Technol. 1996 50 330-342
E. Rytting et al., The AAPS Journal 2005 7(1) E78-E105
G. Piel et al., Int. J. Pharm. 2006 312 75-82

## Claims

1. Method of loading liposomes with a poorly water-soluble substance, said poorly water-soluble substance having an aqueous solubility of less than 1.9 mM at 20 °C and pH = 7, said method comprising:
a) providing a mixture containing:
i. liposomes encapsulating an internal aqueous medium;
ii. said poorly water-soluble substance;
iii. an external aqueous medium optionally comprising sucrose; and
iv. a solubility enhancing agent present in the external aqueous medium, said solubility enhancing agent being selected from the group consisting of complexing agents chosen from α-, β-, and γ-cyclodextrin and cyclodextrins modified with alkyl-, hydroxyalkyl-, dialkyl, and sulphoalkylether groups, and co-solvents chosen from polyethylene glycols (PEGs);
wherein a proton- and/or ion-gradient exists across the liposomal membrane; and
b) incubating said mixture for a period of time sufficient to cause at least part of said poorly water-soluble substance to be drawn out of the external aqueous medium and to accumulate in the liposomes under the influence of said proton and/or ion gradient; and
c) separating the liposomes from the external aqueous medium comprising the remaining poorly water-soluble substance and the solubility enhancing agent.

2. Method according to claim 1, wherein the poorly water-soluble substance has a solubility in water of less than 1 mM at 20 °C and pH = 7.

3. Method according to any one of the preceding claims, wherein the solubility enhancer increases the initial concentration of the poorly-water soluble substance in the external aqueous medium at least two-fold, to values of at least 1.9 mM at 20°C.

4. Method according to claim 1, wherein a pH gradient, a sulphate-, phosphate-, citrate- or acetate-salt gradient, an EDTA-ion gradient, an ammonium-salt gradient, an alkylated ammonium-salt gradient, a Mn²⁺-, Cu²⁺-, Na⁺-, K⁺-gradient, optionally using ionophores in the liposomal membrane, or a combination thereof exists across the liposomal membrane during step b).

5. Method according to any one of the preceding claims, wherein the external aqueous medium comprises saline and/or sucrose and/or EDTA, preferably sucrose and/or EDTA, and the internal aqueous medium comprises poorly water-soluble substance and at least one of a sulphate-, phosphate-, citrate- or acetate-salt, an alkylated ammonium-salt, Mn²⁺-, Cu²⁺-, Na⁺-, or K⁺-ions.

6. Method according to any one of the preceding claims, wherein said poorly water-soluble substance contains a protonizable amine or a carboxyl function or both.

7. Method according to any one of the preceding claims, wherein step b) comprises incubating the mixture for a period of time sufficient to achieve a loading of at least 25%, preferably at least 50% of the total amount of poorly water-soluble substance added to the mixture in step a).

8. Method according to any one of the preceding claims, wherein the separating in step c) comprises dialysis or diafiltration, and preferably comprises dialysis.

9. Method according to any one of the preceding claims, wherein after the steps b) and/or c) the amount of solubility enhancing agent in the internal aqueous medium of the loaded liposomes is lower than 20 mol%, preferably lower than 5 mol% relative to the total amount of the poorly water-soluble substance and solubility enhancing agent.

10. Method according to any one of the preceding claims, wherein after the step c) substantially no solubility enhancing agent is present in the separated liposomal formulation.

11. Method according to any one of the preceding claims, wherein the complexing agent is present in the external aqueous medium in an amount of 0.1-25 w%, more preferably in an amount of 0.5-15 w% and most preferably in an amount of 1-12 w%, based on the total mass of the external aqueous medium, or wherein the co-solvent is present in an amount of at least 1 wt%, more preferably at least 2 wt%, most preferably at least 5 wt%, based on the total mass of the external aqueous medium, and preferably wherein the amount of the co-solvent is less than 75 wt%, preferably less than 50 wt%, more preferably less than 33 wt% and most preferably less than 10 wt%, based on the total mass of the external aqueous medium.

12. Liposome composition, obtainable by the method as defined in any one of the claims 1-11, said composition having a drug to lipid weight ratio of at least 1:7, more preferably at least 1:5, more preferably at least 1:2, wherein the drug is a poorly water-soluble substance having an aqueous solubility of less than 1.9 mM at 20 °C and pH = 7 and wherein the amount of solubility enhancer in the internal aqueous medium of the loaded liposomes is lower than 20 mol% relative to the total amount of the poorly water-soluble substance and solubility enhancer.

13. Liposomal composition according to claim 12, wherein the amount of solubility enhancer in the internal aqueous medium of the loaded liposomes is lower than 5 mol% relative to the total amount of poorly water-soluble substance and solubility enhancer.

## Patentansprüche

1. Verfahren zum Beladen von Liposomen mit einer schlecht wasserlöslichen Substanz, wobei die schlecht wasserlösliche Substanz eine Löslichkeit in Wasser von weniger als 1,9 mM bei 20°C und einem pH-Wert von 7 aufweist, wobei das Verfahren umfasst:
a) Bereitstellen einer Mischung, die enthält:
i. Liposomen, die ein inneres wässriges Medium umschließen;
ii. die schlecht wasserlösliche Substanz;
iii. ein äußeres wässriges Medium, das gegebenenfalls Saccharose enthält; und
iv. einen Löslichkeitsverstärker, der in dem externen wässrigen Medium vorhanden ist und aus der Komplexbildner-Gruppe, ausgewählt aus α-; β- und γ-Cyclodextrin und Cyclodextrinen, die mit Alkyl-; Hydroxyalkyl-; Dialkyl- und Sulfoalkylether-gruppen modifiziert sind, und Co-Lösungsmitteln, ausgewählt aus Polyethylenglykolen (PEGs), ausgewählt ist;
wobei ein Protonen- und/oder Ionen-Gradient durch die liposomale Membran vorhanden ist; und
b) Inkubieren des Gemischs für einen Zeitraum, der ausreicht, um mindestens einen Teil der schlecht wasserlöslichen Substanz aus dem externen wässrigen Medium herausziehen und sich in den Liposomen unter der Einwirkung des Protonen- und/oder Ionengradienten ansammeln zu lassen; und
c) Abtrennen der Liposomen von dem externen wässrigen Medium, das die verbleibende schlecht wasserlösliche Substanz und den Löslichkeitsverstärker enthält.

2. Verfahren nach Anspruch 1, wobei die schlecht wasserlösliche Substanz eine Löslichkeit in Wasser von weniger als 1 mM bei 20°C und einem pH-Wert von 7 aufweist.

3. Verfahren nach einem der vorhergehenden Ansprüche, wobei der Löslichkeitsverstärker die Anfangskonzentration der schlecht wasserlöslichen Substanz in dem externen wässrigen Medium um mindestens das Zweifache erhöht, auf Werte von mindestens 1,9 mM bei 20°C.

4. Verfahren nach Anspruch 1, wobei ein pH-Gradient, ein Sulfat-, Phosphat-, Citrat- oder Acetat-Salz-Gradient, ein EDTA-Ionen-Gradient, ein Ammonium-Salz-Gradient, ein alkylierter Ammonium-Salz-Gradient, ein Mn²⁺-, Cu²⁺-, Na⁺-, K⁺-Gradient, gegebenenfalls unter Verwendung von Ionophoren in der liposomalen Membran, oder eine Kombination davon durch die liposomale Membran während Schritt b) vorhanden ist.

5. Verfahren nach einem der vorhergehenden Ansprüche, **dadurch gekennzeichnet, dass** das äußere wässrige Medium Kochsalzlösung und/oder Saccharose und/oder EDTA, vorzugsweise Saccharose und/oder EDTA, und das innere wässrige Medium eine schlecht wasserlösliche Substanz und mindestens ein Sulfat-, Phosphat-, Citrat- oder Acetat-Salz, ein alkyliertes Ammonium-Salz, Mn²⁺-, Cu²⁺-, Na⁺- oder K⁺-Ionen umfasst.

6. Verfahren nach einem der vorhergehenden Ansprüche, wobei die schlecht wasserlösliche Substanz ein protonierbares Amin oder eine Carboxylfunktion oder beides enthält.

7. Verfahren nach einem der vorhergehenden Ansprüche, wobei Schritt b) das Inkubieren der Mischung für eine ausreichende Zeitspanne umfasst, um eine Beladung von mindestens 25%, vorzugsweise mindestens 50% der Gesamtmenge der schlecht wasserlöslichen Substanz zu erreichen, die der Mischung in Schritt a) zugesetzt wurde.

8. Verfahren nach einem der vorhergehenden Ansprüche, wobei die Abtrennung in Schritt c) Dialyse oder Diafiltration, vorzugsweise Dialyse umfasst.

9. Verfahren nach einem der vorhergehenden Ansprüche, wobei nach den Schritten b) und/oder c) die Menge des Löslichkeitsverstärkers in dem inneren wässrigen Medium der beladenen Liposomen weniger als 20 mol%, vorzugsweise weniger als 5 mol%, bezogen auf die Gesamtmenge der schlecht wasserlöslichen Substanz und des Löslichkeitsverstärkers, ist.

10. Verfahren nach einem der vorhergehenden Ansprüche, wobei nach Schritt c) im Wesentlichen kein Löslichkeitsverstärker in der abgetrennten liposomalen Formulierung vorhanden ist.

11. Verfahren nach einem der vorhergehenden Ansprüche, wobei der Komplexbildner in dem externen wässrigen Medium in einer Menge von 0,1 - 25 Gew.-%, bevorzugter in einer Menge von Menge von 0,5 - 15 Gew.-% und am meisten bevorzugt in einer Menge von 1 - 12 Gew.-%, bezogen auf die Gesamtmasse des externen wässrigen Mediums, oder wobei das Co-Lösungsmittel in einer Menge von mindestens 1 Gew.-%, bevorzugter mindestens 2 Gew.-%, am meisten bevorzugt mindestens 5 Gew.-%, bezogen auf die Gesamtmasse des externen wässrigen Mediums, vorliegt, und
wobei vorzugsweise die Menge des Co-Lösungsmittel weniger als 75 Gew.-%, vorzugsweise weniger als 50 Gew.-%, bevorzugter weniger als 33 Gew.-% und am meisten bevorzugt weniger als 10 Gew.-%, bezogen auf die Gesamtmasse des externen wässrigen Mediums, beträgt.

12. Liposomenzusammensetzung, erhältlich nach dem Verfahren gemäß einem der Ansprüche 1 bis 11, wobei die Zusammensetzung ein Gewichtsverhältnis von Arzneimittel zu Lipid von mindestens 1:7, vorzugsweise mindestens 1:5, bevorzugter mindestens 1:2 aufweist, wobei das Arzneimittel eine schlecht wasserlösliche Substanz mit einer Wasserlöslichkeit von weniger als 1,9 mM bei 20°C und einem pH-Wert von 7 ist und wobei die Menge des Löslichkeitsverstärkers in dem inneren wässrigen Medium der beladenen Liposomen weniger als 20 Mol-% beträgt, bezogen auf die Gesamtmenge der schlecht wasserlöslichen Substanz und des Löslichkeitsverstärkers.

13. Liposomalenzusammensetzung nach Anspruch 12, wobei die Menge des Löslichkeitsverstärkers im inneren wässrigen Medium der beladenen Liposomen weniger als 5 Mol-% beträgt, bezogen auf die der Gesamtmenge der schlecht wasserlöslichen Substanz und des Löslichkeitsverstärkers.

## Revendications

1. - Procédé de chargement dans des liposomes d'une substance médiocrement soluble dans l'eau, ladite substance médiocrement soluble dans l'eau ayant une solubilité dans l'eau de moins de 1,9 mM à 20 °C et pH = 7, ledit procédé comprenant :
a) se procurer un mélange contenant :
i. des liposomes encapsulant un milieu aqueux interne ;
ii. ladite substance médiocrement soluble dans l'eau ;
iii. un milieu aqueux externe facultativement comprenant du saccharose ; et
iv. un agent augmentant la solubilité présent dans le milieu aqueux externe, ledit agent augmentant la solubilité étant choisi dans le groupe consistant en agents complexants choisis parmi l'α-, la β- et la γ-cyclodextrine et les cyclodextrines modifiées par des groupes alkyle, hydroxyalkyle, dialkyle et sulfoalkyléther, et co-solvants choisis parmi les polyéthylène glycols (PEG) ;
un gradient protonique et/ou ionique existant à travers la membrane liposomale ; et
b) faire incuber ledit mélange pendant une période de temps suffisante pour amener au moins une partie de ladite substance médiocrement soluble dans l'eau à être extraite du milieu aqueux externe et à s'accumuler dans les liposomes sous l'influence dudit gradient protonique et/ou ionique ; et
c) séparer les liposomes du milieu aqueux externe comprenant la substance médiocrement soluble dans l'eau restante et l'agent augmentant la solubilité.

2. - Procédé selon la revendication 1, dans lequel la substance médiocrement soluble dans l'eau a une solubilité dans l'eau de moins de 1 mM à 20 °C et pH = 7.

3. - Procédé selon l'une quelconque des revendications précédentes, dans lequel l'agent augmentant la solubilité augmente la concentration initiale de la substance médiocrement soluble dans l'eau dans le milieu aqueux externe d'au moins deux fois, à des valeurs d'au moins 1,9 mM à 20°C.

4. - Procédé selon la revendication 1, dans lequel un gradient de pH, un gradient de sel sulfate, phosphate, citrate ou acétate, un gradient d'ion EDTA, un gradient de sel d'ammonium, un gradient de sel d'ammonium alkylé, un gradient de Mn²⁺, Cu²⁺, Na⁺, K⁺, facultivement à l'aide d'ionophores dans la membrane liposomale, ou une combinaison de ceux-ci, existe à travers la membrane liposomale pendant l'étape b).

5. - Procédé selon l'une quelconque des revendications précédentes, dans lequel le milieu aqueux externe comprend une solution saline et/ou du saccharose et/ou de l'EDTA, de préférence du saccharose et/ou de l'EDTA, et le milieu aqueux interne comprend une substance médiocrement soluble dans l'eau et au moins l'un parmi un sel sulfate, phosphate, citrate ou acétate, un sel d'ammonium alkylé, les ions Mn²⁺, Cu²⁺, Na⁺ ou K⁺.

6. - Procédé selon l'une quelconque des revendications précédentes, dans lequel ladite substance médiocrement soluble dans l'eau contient une amine apte à être protonée ou une fonction carboxyle ou les deux.

7. - Procédé selon l'une quelconque des revendications précédentes, dans lequel l'étape b) comprend l'incubation du mélange pendant une période de temps suffisante pour atteindre un chargement d'au moins 25 %, de préférence d'au moins 50 %, de la quantité totale de substance médiocrement soluble dans l'eau ajoutée au mélange dans l'étape a).

8. - Procédé selon l'une quelconque des revendications précédentes, dans lequel la séparation dans l'étape c) comprend une dialyse ou une diafiltration, et, de préférence, comprend une dialyse.

9. - Procédé selon l'une quelconque des revendications précédentes, dans lequel, après les étapes b) et/ou c), la quantité d'agent augmentant la solubilité dans le milieu aqueux interne des liposomes chargés est inférieure à 20 % en moles, de préférence inférieure à 5 % en moles, par rapport à la quantité totale de la substance médiocrement soluble dans l'eau et de l'agent augmentant la solubilité.

10. - Procédé selon l'une quelconque des revendications précédentes, dans lequel, après l'étape c), sensiblement pas d'agent augmentant la solubilité est présent dans la formulation liposomale séparée.

11. - Procédé selon l'une quelconque des revendications précédentes, dans lequel l'agent complexant est présent dans le milieu aqueux externe dans une quantité de 0,1 à 25 % en poids, de façon davantage préférée dans une quantité de 0,5 à 15 % en poids, et, de la façon que l'on préfère le plus, dans une quantité de 1 à 12 % en poids, sur la base de la masse totale du milieu aqueux externe, ou dans lequel le co-solvant est présent dans une quantité d'au moins 1 % en poids, de façon davantage préférée d'au moins 2 % en poids, de la façon que l'on préfère le plus, d'au moins 5 % en poids, sur la base de la masse totale du milieu aqueux externe, et, de préférence, dans lequel la quantité du co-solvant est inférieure à 75 % en poids, de préférence inférieure à 50 % en poids, de façon davantage préférée inférieure à 33 % en poids et, de la façon que l'on préfère le plus, inférieure à 10 % en poids, sur la base de la masse totale du milieu aqueux externe.

12. - Composition liposomale, susceptible d'être obtenue par le procédé tel que défini dans l'une quelconque des revendications 1 à 11, ladite composition ayant un rapport pondéral médicament à lipide d'au moins 1:7, de façon davantage préférée, d'au moins 1:5, de façon davantage préférée, d'au moins 1:2, le médicament étant une substance médiocrement soluble dans l'eau ayant une solubilité dans l'eau de moins de 1,9 mM à 20 °C et pH = 7, et la quantité d'agent augmentant la solubilité dans le milieu aqueux interne des liposomes chargés étant inférieure à 20 % en moles par rapport à la quantité totale de la substance médiocrement soluble dans l'eau et d'agent augmentant la solubilité.

13. - Composition liposomale selon la revendication 12, dans laquelle la quantité d'agent augmentant la solubilité dans le milieu aqueux interne des liposomes chargés est inférieure à 5 % en moles par rapport à la quantité totale de substance médiocrement soluble dans l'eau et d'agent augmentant la solubilité.
